(19) Europäisches Patentamt
European Patent Office
Office européen des brevets

(11) Veröffentlichungsnummer: **0 024 440**
**B1**

# (12) EUROPÄISCHE PATENTSCHRIFT

(45) Veröffentlichungstag der Patentschrift: **29.06.83**

(51) Int. Cl.³: **C 07 D 251/34**

(21) Anmeldenummer: **79103079.4**

(22) Anmeldetag: **22.08.79**

## (54) Isocyanatgruppenhaltige 2,4,6-Triketohexahydrotriazine und ein Verfahren zu ihrer Herstellung.

(43) Veröffentlichungstag der Anmeldung:
**11.03.81 Patentblatt 81/10**

(45) Bekanntmachung des Hinweises auf die Patenterteilung:
**29.06.83 Patentblatt 83/26**

(84) Benannte Vertragsstaaten:
**AT BE CH DE FR GB IT LU NL SE**

(56) Entgegenhaltungen:
**EP - A - 0 013 880**
**DE - A - 2 325 826**
**DE - A - 2 644 684**

**JOURNAL OF POLYMER SCIENCE: Polymer Chemistry Edition, Band 12, 1974, Seiten 455—468 New York, USA H. MATSUDA: "Synthesis of polymers by using divalent metal salts of mono(hydroxyethyl)phthalate:metal-containing polyurethanes"**

(73) Patentinhaber: **CHEMISCHE WERKE HÜLS AG**
**Postfach 1320**
**D-4370 Marl 1 (DE)**

(72) Erfinder: **Disteldorf, Josef, Dr.**
**Am Sengenhoff 2a**
**D-4690 Herne 1 (DE)**
Erfinder: **Hübel, Werner, Dr.**
**Birnenbruchstrasse 34**
**D-4690 Herne 1 (DE)**
Erfinder: **Wolf, Elmar, Dr.**
**Am Böckenbusch 3a**
**D-4690 Herne 2 (DE)**

(74) Vertreter: **Steil, Hanna, Dipl.-Chem.**
**RSP PATENTE - PB 15 Postfach 1320**
**D-4370 Marl 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 024 440 B1

# Isocyanatgruppenhaltige 2,4,6-Triketohexahydrotriazine und ein Verfahren zu ihrer Herstellung

Die Trimerisierung von organischen Isocyanaten ist eine bekannte Reaktion. Als Katalysatoren für die Trimerisierung von organischen Isocyanaten werden in der Literatur eine Vielzahl von chemisch sehr verschiedenartigen Verbindungen beschrieben. So können als Trimerisierungskatalysatoren Metallverbindungen aus der Gruppe Salze, Basen und homöopolare Metallverbindungen, wie Metallnaphthenate, Na-Benzoat in Dimethylformamid (DMF), Erdalkaliacetate, -formiate und -carbonate, Metallalkoxide, $AlCl_3$ und Fe-Acetylacetonat eingesetzt werden. Diese lassen sich aus den Reaktionsprodukten, den Isocyanuraten, nur sehr schwierig beziehungsweise schlecht oder überhaupt nicht abtrennen. Sie verbleiben vielmehr im Reaktionsprodukt.

Dieses ist natürlich von großem Nachteil, vor allem dann, wenn es sich um ein Isocyanurat handelt, das durch Trimerisierung eines Diisocyanats hergestellt worden ist. Beim Erhitzen derartiger Produkte reagieren die freien NCO-Gruppen dieser Isocyanurate in Gegenwart des Trimerisierungskatalysators noch weiter. Es bilden sich höhermolekulare, Isocyanuratringe- enthaltende Polyisocyanate. Mit zunehmender Reaktion tritt sogar Vernetzung ein. Aus diesem Grunde ist es auch verständlich, daß noch katalysatorhaltige Isocyanurate, die noch über Alkylengruppen am Triazinring gebundene NCO-Gruppen enthalten, kaum zur gezielten Weiterreaktion mit Alkoholen oder Aminen eingesetzt werden können.

Das Ziel bei jeglicher Herstellung von Isocyanuraten (aus Polyisocyanaten) muß daher die vollständige Entfernung bzw. Desaktivierung des Katalysators nach beendeter Trimerisierung sein. Dieses läßt sich einmal realisieren, indem man leichtflüchtige, basische Verbindungen, wie tertiäre Amine oder Phosphine, als Trimerisierungskatalysator einsetzt. Der Nachteil bei Verwendung dieser Verbindungen besteht allerdings darin, daß sie (tertiäre Phosphine) nicht nur die Trimerisierung, sondern auch z.T. die Dimerisierung der Isocyanate katalysieren. Weiterhin muß darauf hingewiesen werden, daß tertiäre Amine sehr gut die Trimerisierung von aromatischen Isocyanaten katalysieren, bei aliphatischen oder cycloaliphatischen Isocyanaten dagegen keinen katalytischen Effekt zeigen. So gelingt beispielsweise eine Trimerisierung von Hexamethylendiisocyanat (HDI), 2,2,4- bzw. (2,4,4)-Trimethylhexamethylendiisocyanat- (1,6) (TMDI); 3-Isocyanatomethyl-3,5,5-Trimethyl-cyclohexylisocyanat, genannt auch Isophorondiisocyanat (IPDI), 3, bzw. (4) 8, bzw. (9)-Diisocyanatomethyl-tricyclo-[5.2.1.$0^{2,6}$]-decan (TCDI), Diisocyanatomethylnorbornan (NDI) weder mit Triäthylamin, Dimethylbenzylamin noch mit 1,4-Diazabicyclo[2.2.2]octan. Diese aliphatischen bzw. cycloaliphatischen Isocyanate lassen dagegen mit Na-Benzoat in DMF (Chem. Abstr. *60*, 8332 (1963). oder mit Na-Phenolaten in n-Butylacetat (GB—PS 1 386 399) oder mit Aziridin (DE—OS 23 25 268; IPDI; TMDI) oder mit speziellen Epoxidverbindungen (DE—OS 26 44 684; IPDI, TMDI) gut trimerisieren. Der große Nachteil dieser eben genannten Verfahren besteht in ihrer Katalysatorabtrennung vom Reaktionsprodukt. Na-Benzoat wie auch Na-Phenolat müssen nach beendeter Trimerisierung des Diisocyanats vom Reaktionsprodukt abfiltriert werden. Diese Filtration bereitet insofern Schwierigkeiten, da es sich um sehr fein kristalline (Na-Benzoat Teilchengröße $< 2\ \mu$) beziehungsweise schleimartige Niederschläge (Na-Phenolat) handelt. Im übrigen sind 2,4,6-Triketohexahydrotriazine auf Basis eines Methylnonandiisocyanates bekannt.

Es wurde nun überraschenderweise gefunden, daß diese Schwierigkeiten der Katalysatorabtrennung, wie es beispielsweise bei der Trimerisierung mit Na-Benzoat und Na-phenolat der Fall ist, nicht auftreten, wenn man das Verfahren zur Herstellung von 2,4,6-Triketohexahydrotriazinen durch katalytische Trimerisierung von organischen Isocyanaten bei erhöhter Temperatur so durchführt, daß man die Trimerisierung in Anwesenheit eines Katalysators der allgemeinen Formel

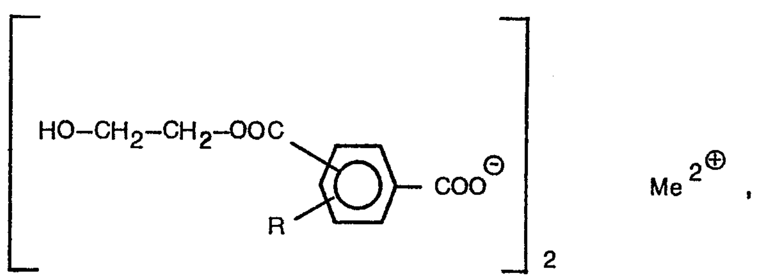

in der R Wasserstoff- oder ein $C_1$—$C_4$-Alkylrest und Me ein zweiwertiges Metallkation ist, und deren hydrierte Derivate in einer Menge von 0,1—5 Gew.%, vorzugsweise 0,5—3 Gew.%, und in einem inerten, polaren Lösungsmittel in einer Menge von 5—40 Gew.%, vorzugsweise 10—20 Gew.%, jeweils bezogen auf das eingesetzte Isocyanat, bei Temperaturen von 60—150°C, vorzugsweise 80—130°C, durchführt und die Reaktion bei einem Umsatz von etwa 50% durch Kühlung abbricht und anschließend das entstandene Isocyanurat auf bekannte Weise isoliert.

Die erfindungsgemäß verwendeten Katalysatoren und deren Herstellung sind bekannt. Sie werden in der Literatur als Reaktionspartner von Diisocyanaten, nämlich als hydroxylgruppenhaltige

Komponente, zur Herstellung (in DMF) von "metal-containing polyurethanes" (H. Matsuda, J. Polym. Sci. *12.* (1974) 455—468) beschrieben. Es war deshalb umso erstaunlicher, daß diese Verbindungen in erheblich geringerer Konzentration die Trimerisierung von Isocyanaten zu katalysieren vermögen.

Als Ausgangsmaterial für das erfindungsgemäße Verfahren sind alle Mono- und Polyisocyanate geeignet, wie aliphatische, cycloaliphatische, araliphatische, arylsubstituierte aliphatische und/oder aromatische Diisocyanate, wie sie beispielsweise in Houben-Weyl, Methoden der organischen Chemie, Band 14/2, S. 61—70 und dem Artikel von W. Siefken in Justus Liebigs Annalen der Chemie *562*,75—136, beschrieben werden. Verwendet man Di- oder andere Polyisocyanate, so kann man entsprechend isocyanatgruppenhaltige Isocyanurate erhalten, die von erheblicher technischer Bedeutung sind. So sind beispielsweise aliphatische Isocyanurate aus Diisocyanaten in Kombination mit hydroxylgruppenhaltigen Verbindungen zur Herstellung von klaren und pigmentierten Polyurethan-Zweikomponentenlacken, die sich durch schnelle Trocknung und gute Lichtstabilität auszeichnen, hervorragend geeignet.

Geeignete Isocyanate sind beispielsweise die Monoisocyanate, wie Methylisocyanat, Äthylisocyanat, Propylisocyanat, n-Butylisocyanat, Isohexylisocyanat, Dodecyl-isocyanat, Oleylisocyanat, Stearylisocyanat, Cyclohexylisocyanat, 2 - Chloräthylisocyanat, 2 - Cyanäthylisocyanat, 6 - Chlorhexylisocyanat, Isocyanatoessigsäureäthylester, Propylätherpropylisocyanat, Benzylisocyanat, Phenyläthylisocyanat, u.a., die Diisocyanate, wie Methandiisocyanat, Butan - 1,1 - diisocyanat, 1,2 - Aethylendiisocyanat, Butan - 1,2 - diisocyanat, Transvinylendiisocyanat, Propan - 1,3 - diisocyanat, 1,4 - Tetramethylendiisocyanat, 2 - Buten - 1,4 - diisocyanat, 2 - Methylbutan - 1,4 - diisocyanat, Pentan - 1,5 - diisocyanat, 2,2 - Dimethylpentan - 1,5 - diisocyanat, 1,6 - Hexamethylendiisocyanat, 1,1′ - Di - naphthyl - 2,2′ - diisocyanat, 2,2,4 - bzw. 2,4,4 - Trimethyl - hexamethylendiisocyanat - 1,6 (TMDI), Heptan - 1,7 - diisocyanat, Octan - 1,8 - diisocyanat, Nonan - 1,9 - diisocyanat, Decan - 1,10 - diisocyanat, 1,12 - Dodecandiisocyanat, $\omega,\omega'$ - Diisocyanatodi - n - propyläther, Dimethylsilandiisocyanat, Diphenylsilandiisocyanat, die Ester von 2,6 - Diisocyanatcapronsäure, wie Methyl-, Methoxymethyl-, 1,2 - Dichloropropyl-, Isopropylester; die entsprechenden Diester von 2,4 - Diisocyanatoglutarsäure, 2,5 - Diisocyanatoadipinsäure, 2,6 - Diisocyanatopimelinsäure, 2,7 - Diisocyanatokorksäure, 2,9 - Diisocyanatosebacinsäure, Cyclobutan - 1,3 - diisocyanat, Di - (isocyanatmethyl) - cyclobutan, Cyclohexan - 1,3- und 1,4 - diisocyanat, $\omega,\omega'$ - 1,3 - Dimethylcyclohexandiisocyanat, $\omega,\omega'$ - 1,4 - Dimethylcyclohexandiisocyanat, 3 - Isocyanatomethyl - 3,5,5 - trimethylcyclohexylisocyanat, welches auch als Isophorondiisocyanat bezeichnet und mit IPDI abgekürzt wird, Decahydro - 8 - methyl - (1,4 - methano - naphthalen - 2 (oder 3) 5 - ylendimethylen - diisocyanat, Decahydro - 4,7 - methano - indan - 1 (oder 2) 5 (oder 6) ylendimethylendiisocyanat, Hexahydro - 4 - 7 - methanindan - 1 - (oder 2) 5 (oder 6) - ylendiisocyanat, Hexahydro - 1,3 - bzw. - 1,4 - phenylen - diisocyanat, 2,4 - und 2,6 - Hexahydrotoluylendiisocyanat, hydriertes Diphenyläthandiisocyanat, hydriertes Diphenylpropandiisocyanat, hydriertes Diphenylbutandiisocyanat, $\omega,\omega'$, - Diisocyanato - 1,4 - diäthylbenzol, 1,3 - bzw. 1,4 - Phenylendiisocyanat, 1 - Methylbenzol - 2,4 - diisocyanat, 1 - Methylbenzol - 2,5 - diisocyanat, 1 - Methylbenzol - 2,6 - diisocyanat, 1 - Methylbenzol - 3,5 - diisocyanat, $\omega,\omega'$, - 1,3 - Dimethylbenzoldiisocyanat, $\omega,\omega'$, - 1,4 - Dimethylcyclohexandiisocyanat, 1 - Isopropylbenzol - 2,4 - diisocyanat, 1 - Chlorbenzol - 2,4 - diisocyanat, 1 - Fluorbenzol - 2,4 - diisocyanat, 1 - Nitrobenzol - 2,4 - diisocyanat, 1 - Chlor - 4 - methoxybenzol - 2,5 - diisocyanat, Azobenzol - 4,4′ - diisocyanat, Benzolazonaphthalin - 4,4′ - diisocyanat, 2,4′ - 4,4′ - Diisocyanato - diphenyl, Diphenyläther - 4,4′ - diisocyanat, Diphenyläther - 2,4 - diisocyanat, 4,4′ - Diisocyanato - 3,3′ - dichlor - diphenyl, 4,4′ - Diisocyanato - 2,3′ - bzw. 3,3′ - dimethoxy - diphenyl, 4,4′ - Diisocyanato - 3,3′ - dimethyl - diphenyl, 4,4′ - Diisocyanato - 3,3′ - diphenyl - diphenyl, 4,4′ - Diisocyanato - diphenylmethan, 3,3′ - Dimethoxydiphenylmethan - 4,4′ - diisocyanat, 4,4′ - Dimethoxyphenylmethan - 3,3′ - diisocyanat, Naphthalin - 1,4-, 1,5- bzw. 3,5 - diisocyanat, $\omega,\omega'$ - 1,4 - Dimethylnaphthalindiisocyanat, $\omega,\omega,'$ - 1,5 - Dimethylnaphthalindiisocyanat, 1,1′ - Di - naphthyl - 2,2′ - diisocyanat, Toluoldiisocyante, Toluol - 2,4- bzw. 2,6 - diisocyanat, Diphenylsulfid - 4,4′ - diisocyanat, Diphenylsulfon - 4,4′ - diisocyanat, Benzophenon - 3,3′ - diisocyanat, Fluoren - 2,7 - diisocyanat, Anthrachinon - 2,6 - diisocyanat, Pyren - 3,8 - diisocyanat, Chrysen - 2,8 - diisocyanat, N,N′ - (4,4′ - Dimethyl - 3,3′ - diisocyanatodiphenyl) - uretdion, m - bzw. p - Xylylen - diisocyanat, aber auch die Triisocyanate, wie 1,6,11 - Triisocyanatoundecan, 2,4,4′ - Triisocyanato - diphenyläther, 4,4′, 4″ - Trisocyanatotriphenylmethan, Tris - (4 - isocyanatophenyl) - thiophosphat, sowie beliebige Gemische dieser Isomeren. Weitere geeignete Isocyanate werden in dem genannten Artikel in den Annalen auf Seite 122 f. beschrieben. Es können auch Gemische der vorgenannten Isocyanate eingesetzt werden.

Besonders bevorzugt werden in der Regel die technisch leicht zugängigen aliphatischen, cycloaliphatischen oder aromatischen Diisocyanate und besonders das 3-Isocyanatomethyl-3,5,5-trimethylcyclohexylisocyanat und 2,4-Toluylendiisocyanat sowie deren isomere Gemische.

Die erfindungsgemäßen eingesetzten Katalysatoren werden in Mengen von 0,1—5 Gew.-%, vorzugsweise 0,5—3 Gew.-%, bezogen auf die Menge des eingesetzten Isocyanats eingesetzt. Voraussetzung für die Wirksamkeit des Katalysators ist natürlich vollständige oder wenigstens teilweise Löslichkeit in einem organischen Lösungsmittel, der verständlicherweise auch in dem zu trimerisierenden Isocyanat löslich ist.

Die Herstellung des erfindungsgemäßen Katalysators kann beispielsweise nach den in J. Polym. Sci. 12, 456 (1974) gemachten Angaben erfolgen. So wird beispielsweise Phthalsäureanhydrid in Gegenwart eines Katalysators aus der Gruppe der tertiären Amine, z.B. (N,N-Dimethylbenzylamin) mit Äthylenglykol in einem 1. Reaktionsschritt zum Mono-(hydroxyäthyl)-phthalat umgesetzt. In einem weiteren Reaktionsschritt wird dann der saure Ester, das Mono-(hydroxyäthylphthalat), beispielsweise mit dem Erdalkalioxid zum entsprechenden Erdalkali- (monohydroxyäthyl)-phthalat neutralisiert. Unter zweiwertigen Metallkationen werden die der Erdalkalimetalle, wie Magnesium und Calcium, sowie Zink und Zinn verstanden.

Als Lösungsmittel kommen vor allem stark polare in Frage, wie Dimethylformamid, Dimethylsulfoxid oder auch hexamethylphosphorsäuretriamid. Je höher die Löslichkeit des Katalysators im Lösungsmittel, desto geringer ist die Konzentration des Lösungsmittels (bezogen auf eingesetztes Isocyanat). Es wird in Mengen von 5—40 Gew.%, vorzugsweise 10—20 Gew.-%, eingesetzt.

Zur Herstellung der Isocyanurate nach dem erfindungsgemäßen Verfahren legt man das zu trimerisierende Isocyanat vor, gibt das Lösungsmittel in Mengen von 5—40 Gew.-%, vorzugsweise 10—20 Gew.-% und den Katalysator in Mengen von 0,1—5 Gew.-%, vorzugsweise 0,5—3 Gew.-% zu, und erwärmt das Gemisch auf eine Temperatur zwischen 60 und 150°C, vorzugsweise 80—130°C. Die Umsetzungsgeschwindigkeit des Isocyanats zum Isocyanurat wird durch Bestimmung des NCO-Gehalts im Reaktionsgemisch kontrolliert.

Für die Herstellung des monomerfreien Isocyanurats hat es sich als zweckmäßig erwiesen, das Isocyanat nur teilweise zu trimerisieren, d.h. nach einem Umsatz von ca. 50% die Reaktion durch Kühlung auf Raum-temperatur abzubrechen, und in einem 2. Reaktionsschritt das freie Isocyanat und das Lösungsmittel durch Dünnschichtdestillation vom Isocyanurat abzutrennen.

Liegen die Siedepunkte des Isocyanats und des Lösungsmittels sehr weit auseinander, so ist es für das erfindungsgemäße Verfahren sehr vorteilhaft, die beiden Komponenten einzeln abzutrennen. Dies geschieht in einer mehrstufigen Dünnschichtverdampfung. Das abdestillierte Isocyanat sowie das abdestillierte Lösungsmittel können wieder zur Trimerisierung eingesetzt werden.

Gegenstad der Erfindung sind isocyanathaltige 2,4,6-Triketohexyhydrotriazine der folgenden Struktur:

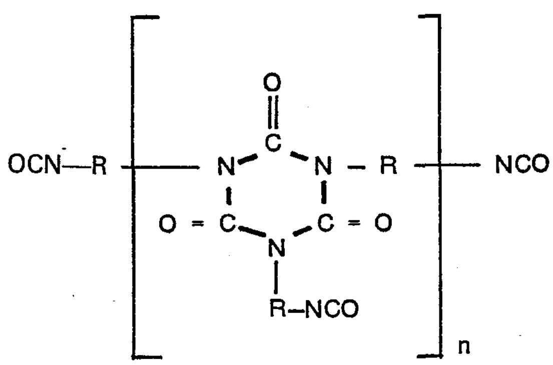

wobei R:

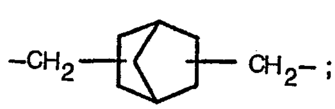

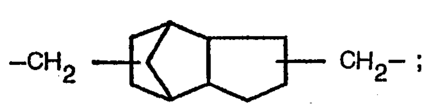

und n eine ganze Zahl, vorzugsweise 1—5, bedeutet,

Diese Isocyanurate sind überraschenderweise in der Literatur noch nicht beschrieben.

Weiterer Gegenstand der Erfindung ist ein Verfahren zur herstellung der vorstehend angeführten isocyanatgruppenhaltigen Triketohexahydrotriazine durch Trimerisierung in Anwesenheit eines Katalysators der allgemeinen Formel

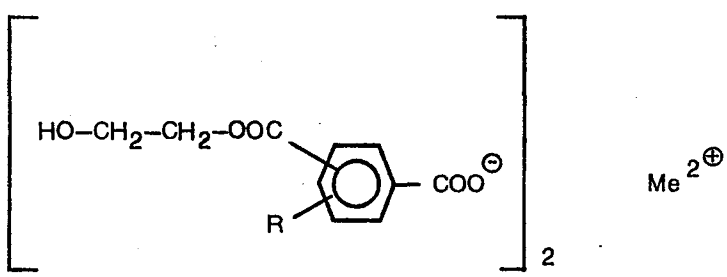

,

in der R Wasserstoff- oder ein $C_1$—$C_4$-Alkylrest und Me ein zweiwertiges Metallkation ist, und deren hydrierte Derivate in einer Menge von 0,1—5 Gew.%, und in einem inerten, polaren Lösungsmittel in einer Menge von 5—40 Gew.%, jeweils bezogen auf das eingesetzte Isocyanate, bei Temperaturen von 60—150°C durchführt und die Reaktion bei einem Umsatz von etwa 50% durch Kühlung abbricht und anschließend das entstandene Isocyanat auf bekannte Weise isoliert.

Die nach dem erfindungsgemäßen Verfahren hergestellten Isocyanatoisocyanurate aus Diisocyanaten sind in den üblichen Lacklösungsmitteln, wie Xylol, Äthylglykolacetat, Butylacetaten usw., gut löslich. Diese Lösungen des Isocyanatoisocyanurats sind klar und verändern sich auch beim Lagern nicht. Die Isocyanurate stellen wertvolle Vorprodukte dar.

Das Verfahren der vorliegenden Erfindung wird durch die nachstehenden Beispiele illustriert:

A. Herstellung des Katalysators

1. Zu 248,3 Gew.T (4 Mol) Äthylenglykol und 1,14 Gew.T N,N-Dimethylbenzylamin wurden bei 70°C innerhalb 2 Stunden 118,5 Gew.T (0,8 Mol) Phthalsäureanhydrid zugegeben. Nach der Phthalsäureanhydridzugabe wurde das Reaktionsgemisch noch weitere 2 Stunden bei 70°C erhitzt. Danach wurde abgekühlt und 90 Gew.T Aceton zugegeben. Zu dieser Lösung wurden bei Raumtemperatur unter intensiver Rührung innerhalb ca. 30 Minuten 15,3 Gew.T MgO zugegeben. Nach Beendigung der MgO-Zugabe wurde das Reaktionsgemisch noch weitere 20 Minuten bei Raumtemperatur und anschließend 2 Stunden bei 60°C erhitzt. Es bildete sich ein weißer Niederschlag, der abfiltriert, mehrere Male mit Aceton gewaschen und anschließend im Vakuumtrockenschrank bei 60°C getrocknet wird.

Die Ausbeute betrug 70% bezogen auf eingesetztes Phthalsäureanhydrid.

2. Die Herstellung anderer Salze zweiwertiger Metallkationen der Monohydroxyläthyl-phthalsäuren und deren hydrierte Derivate kann in Analogie dazu erfolgen.

B. Trimerisierung von Isocyanaten nach dem erfindungsgemäßen Verfahren

Beispiel 1

500 Gew.T TCDI, 2,5 Gew.T. Ca-Salze des Monohydroxyäthylphthalats als Katalysator, hergestellt gemäß Vorschrift $A_1$, und 100 Gew.T Dimethylformamid (DMF) wurden gemischt und auf 130°C erhitzt. Nach 1 Stunde betrug der NCO-Gehalt der Reaktionslösung 21,6%. Darauf wurde rasch auf 60°C abgekühlt. Bei dieser Temperatur wurde im Rotationsverdampfer (bei ca. 133 Pa) das Reaktionsgemisch weitgehend vom DMF befreit. Zur Entfernung des nicht umgesetzten TCDI und der geringen Restmenge an DMF wurde das Reaktionsgemisch bei 210°C/6,7 Pa im Dünnschichtverdampfer destilliert. Der Rückstand hatte einen NCO-Gehalt von 11,3% und einen Monomergehalt von < 0,5. Die Viskosität des Reaktionsproduktes betrug bei 120°C 400 Pa.s, bei 140°C 100 Pa.s.

Das IR-Spektrum des Reaktionsproduktes zeigt die Abb. 1.

Beispiel 2

500 Gew.T TCDI, 3 Gew.T. Katalysator des Ca-Salzes entsprechend Vorschrift $A_1$ und 30 Gew.T. DMF wurden bei Raumtemperatur zusammengegeben und anschließend auf 120°C erhitzt. Nach 50 Minuten betrug der NCO-Gehalt der Reaktionslösung 23%. Daraufhin wurde die Reaktionslösung rasch auf 60°C abgekühlt und dann so aufgearbeitet, wie im Beispiel $B_1$ beschrieben.

Das Reaktionsprodukt (Rückstand der Dünnschichtdestillation) hatte einen NCO-Gehalt von 11,6% und einen Monomerengehalt von < 0,6%. Seine Viskosität bei 120°C betrug 380 Pa·s, bei 140°C 96 Pa·s.

Beispiel 3

1 000 Gew.T. IPDI, 200 Gew.T. DMF und 1 Gew.T. Katalysator gemäß $A_1$ wurden gemischt und anschließend auf 130°C erhitzt. Nach 3-stündigem Erhitzen auf 130°C betrug der NCO-Gehalt der Reaktionslösung 17,8%. Nach Erreichen dieses NCO-Gehaltes wurde das Reaktionsgemisch auf 60°C abgekühlt und, wie in Beispiel 1 + 2 bereits beschrieben, vom Lösungsmittel (DMF) befreit. Das nahezu DMF-freie IPDI-haltige IPDI-Isocyanuratgemisch wurde dann bei 190°C/6,7 Pa der Dünnschichtverdampfung zugeführt. Der Destillationsrückstand (Reaktionsprodukt) hatte einen NCO-Gehalt von 17,4%, einen Monomerengehalt von < 0,5% und eine Viskosität bei 120°C von 140 Pa·s, bei 140°C von 41 Pa·s.

Das Reaktionsprodukt (% NCO: 17,4) ließ sich in Xylol-Gemisch/Äthylglykolacetat zu einer klaren 70 Gew.%-igen Lösung mit einer Viskosität von 1,35 Pa bei 25°C lösen.

Beispiel 4

500 Gew.T NDI, 130 Gew.T DMF und 5 Gew.T. Katalysator gemäß Vorschrift $A_1$ wurden gemischt und anschließend auf 120°C erhitzt. Nach 1-stündigem Erhitzen auf 120°C betrug der NCO-Gehalt der Reaktionslösung 24,3%. Nach Erreichen dieses NCO-Gehaltes wurde das Reaktionsgemisch so wie in den Beispielen $B_{1-3}$ weiterbehandelt. Das Reaktionsprodukt (Rückstand der Dünnschichtdestillation) enthielt einen NCO-Gehalt von 15,3% und einen Monomerengehalt von < 0,7%. Seine Viskosität bei 120°C betrug 160 Pa·s und bei 140°C 63 Pa·s.

Beispiel 5

500 Gew.T. TMDI, 100 Gew.T DMF und 2 Gew.T. Katalysator des Ca-Salzes entsprechend Vorschrift $A_1$ wurden gemischt und anschließend auf 120°C erhitzt. Nach 45-minütigem Erhitzen auf 120°C betrug der NCO-Gehalt der Reaktionslösung 26,7%. Nach Erreichen dieses NCO-Gehaltes wurde das Reaktionsgemisch so wie in den Beispielen 1—4 weiterbehandelt. Das Reaktionsprodukt (Rückstand der Dünnschichtdestillation) enthielt einen NCO-Gehalt von 16,7% und einen Monomergehalt von < 0,6%. Seine Viskosität bei Raumtemperatur betrug 540 Pa·s, bei 40°C 90 Pa·s, bei 60°C 12 Pa·s, bei 80°C 3,3 Pa·s, bei 100°C 0,2 Pa·s.

Beispiel 6

200 Gew.T. eines Isomeren-Gemisches aus 30 Gew.T. Toluylen-2.4-diisocyanat und 20 Gew.T. Toluylen-2.6-diisocyanat, 30 Gew.T. DMF und 0,4 Gew.T. Katalysator gemäß Beispiel $A_1$ wurden gemischt und 30 Minuten auf 80°C erhitzt. Nach dieser Zeit betrug der NCO-Gehalt dieser Lösung 26,6%, das bedeutet, daß ca. 75% des Isocyanats unter Trimerisierung reagiert haben. Die Viskositat dieser Lösung betrug bei 25°C 0,61 Pa·s.

Beispiel 7

1000 Gew.T. 5-Methyl-nonamethylendiisocyanat-1,9 (MNDI), 200 Gew.T. DMF und 2 Gew.T. Katalysator gemäß $A_1$ wurden gemischt und anschließend auf 130°C erhitzt. Nach ca. 3-stündigem Erhitzen bei 130°C betrug der NCO-Gehalt der Reaktionslösung 18,5%. Nach Erreichen dieses NCO-Gehalts wurde das Reaktionsgemisch auf 60°C abgekühlt und, wie in Beispiel 1—3 bereits beschrieben, von Lösungsmittel (DMF) befreit. Das nahezu DMF-freie MNDI-haltige MNDI-Isocyanuratgemisch wurde dann bei 190°C/6,7 Pa der Dünnsohichtverdampfung zugeführt. Das Reaktionsprodukt (Destillationsrückstand) hatte einen NCO-Gehalt von 15,4%, einen Monomerengehalt von < 0,7% und eine Viskosität bei 25°C von 5,7 Pa·s, bei 40°C von 1,8 Pa·s und bei 60°C von 0,6 Pa·s.

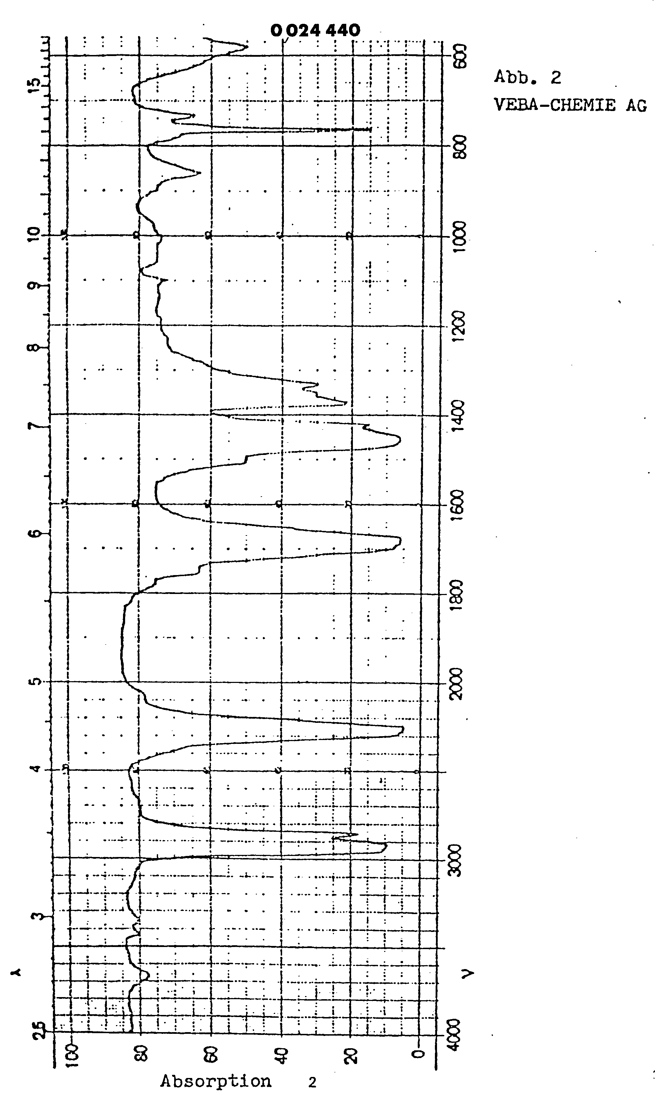

Das IR-Spektrum des Reaktionsproduktes ziegt Abb. 2.

**Patentansprüche**

1. Isocyanatgruppenhaltige 2,4,6-Triketohexahydrotriazine der folgenden Struktur

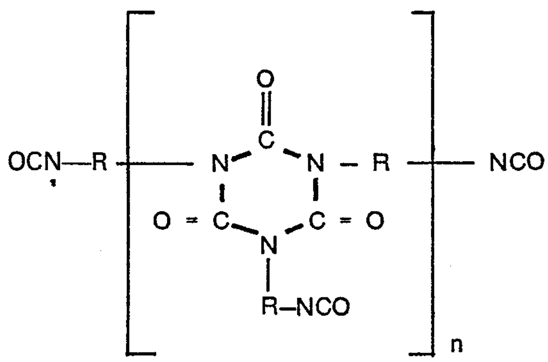

worin R ein zweiwertiger Rest aus der Gruppe

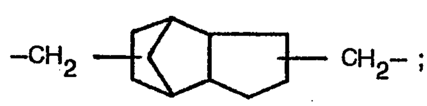 ; 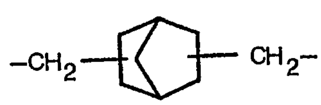 ;

und n = 1—5 ist.

2. Verfahren zur Herstellung von isocyanatgruppenhaltigen 2,4,6-Triketohexahydrotriazinen gemäß Anspruch 1 durch katalytische Trimerisierung von organischen Isocyanaten bei erhöhter Temperatur, dadurch gekennzeichnet, daß man die Trimerisierung in Anwesenheit eines Katalysators der allgemeinen Formel

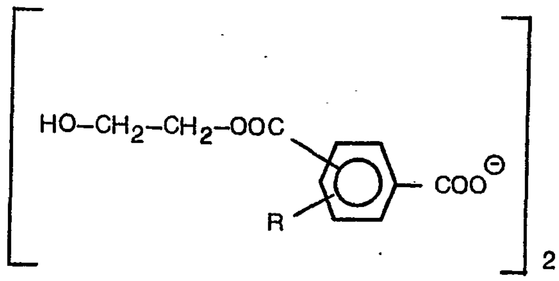 $Me^{2\oplus}$ ,

in der R Wasserstoff- oder ein $C_1$—$C_4$-Alkylrest und Me ein zweiwertiges Metallkation ist, und deren hydrierte Derivate in einer Menge von 0,1—5 Gew.%, und in einem inerten, polaren Lösungsmittel in einer Menge von 5—40 Gew.%, jeweils bezogen auf das eingesetzte Isocyanat, bei Temperaturen von 60—150°C durchführt und die Reaktion bei einem Umsatz von etwa 50% durch Kühlung abbricht und anschließend das entstandene 2,4,6-Triketohexahydrotriazin auf bekannte Weise isoliert.

3. Verfahren nach Anspruch 2, dadurch gekennzeichnet, daß man die Trimerisierung bei Temperaturen im Bereich von 80—130°C durchführt.

4. Verfahren nach den Ansprüchen 2 oder 3, dadurch gekennzeichnet, daß man das nicht umgesetzte Isocyanat sowie das Lösungsmittel durch Dünnschichtdestillation abtrennt.

**Revendications**

1. 2,4,6-tricétohexahydrotriazines, contenant des groupes isocyanate, et ayant la structure suivante:

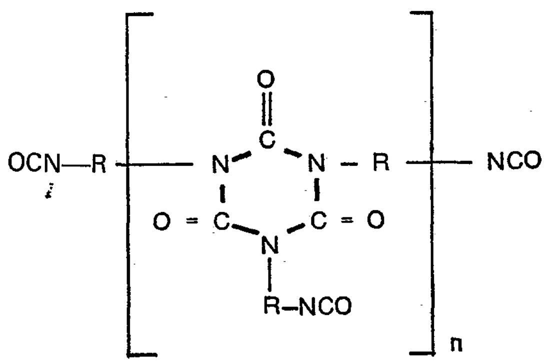

dans laquelle:

R est un reste bivalent, du groupe

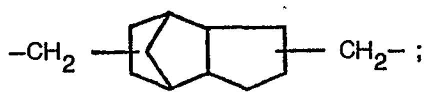 ; 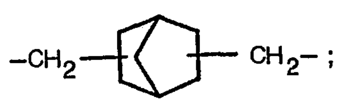 ;

et n = 1—5.

2. Procédé pour la préparation de 2,4,6-tricétohexahydrotriazines, contenant des groupes isocyanate, selon la revendication 1, par trimérisation catalytique d'isocyanates organiques à température élevée, procédé caractérisé en ce que la trimérisation est effectuée en présence d'un catalyseur de formule générale:

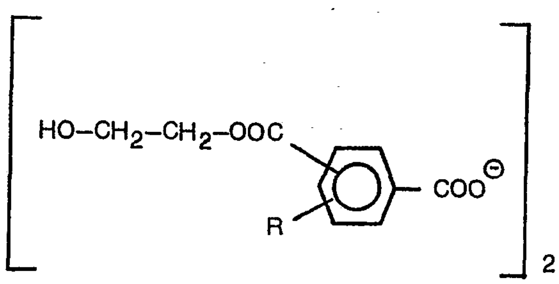 $Me^{2\oplus}$ ,

où R représente un hydrogène ou un reste alcoyle en $C_1$—$C_4$, et Me est un cation métallique bivalent, et son dérivé hydrogéné, dans une proportion de 0,1 à 5% en poids et dans un solvant polaire, inerte, dans une proportion de 5 à 40% en poids, rapporté chaque fois à 1 isocyanate mis en oeuvre, à des

températures de 60—150°C, et que la réaction est stoppée par refroidissement quand le taux de transformation a atteint 50% et qu'ensuite, le 2,4,6-tricétohexahydrotriazine obtenu est isolé d'une manière connue.

3. Procédé selon la revendication 2, caractérisé en ce que la trimérisation est effectuée à des températures de 80—130°C.

4. Procédé selon l'une des revendications 2 ou 3, caractérisé en ce que l'isocyanate non réagi, et le solvant, sont éliminés par distillation en couche mince.

**Claims**

1. 2,4,6-Triketohexahydrotriazines containing isocyanate groups and having the following structure

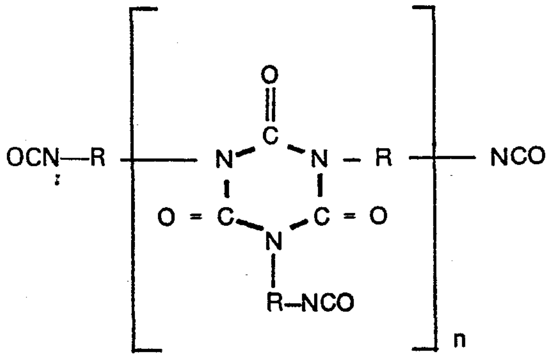

wherein R is a divalent radical from the group comprising

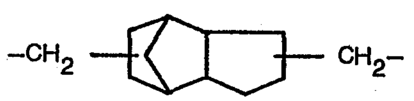 ; 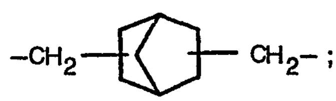 ;

and n is 1—5.

2. Process for the preparation of 2,4,6-triketohexahydrotriazines containing isocyanate groups, according to Claim 1, by catalytic trimerisation of organic isocyanates at elevated temperature, characterised in that the trimerisation is carried out in the presence of a catalyst of the general formula

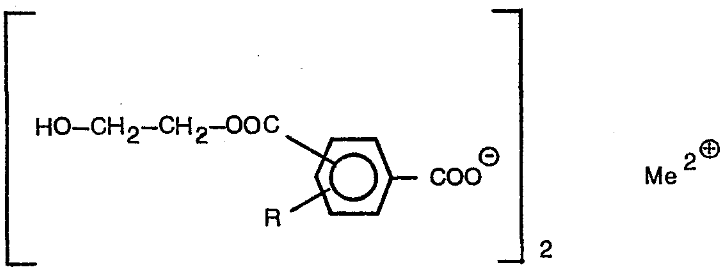 ,

in which R is hydrogen or a $C_1$—$C_4$-alkyl radical and Me is a divalent metal cation, and hydrogenated derivatives thereof, in an amount of 0.1—5% by weight, and in an inert, polar solvent in an amount of 5—40% by weight, in each case based on the isocyanate employed, at temperatures of 60—150°C, and the reaction is interrupted at a conversion of about 50% by cooling, and the 2,4,6-triketohexahydrotriazine formed is then isolated in a known manner.

3. Process according to Claim 2, characterised in that the trimerisation is carried out at temperatures in the range of 80—130°C.

4. Process according to Claims 2 or 3, characterised in that the unreacted isocyanate and the solvent are separated off by thin film distillation.

Abb. 1

VEBA-CHEMIE AG

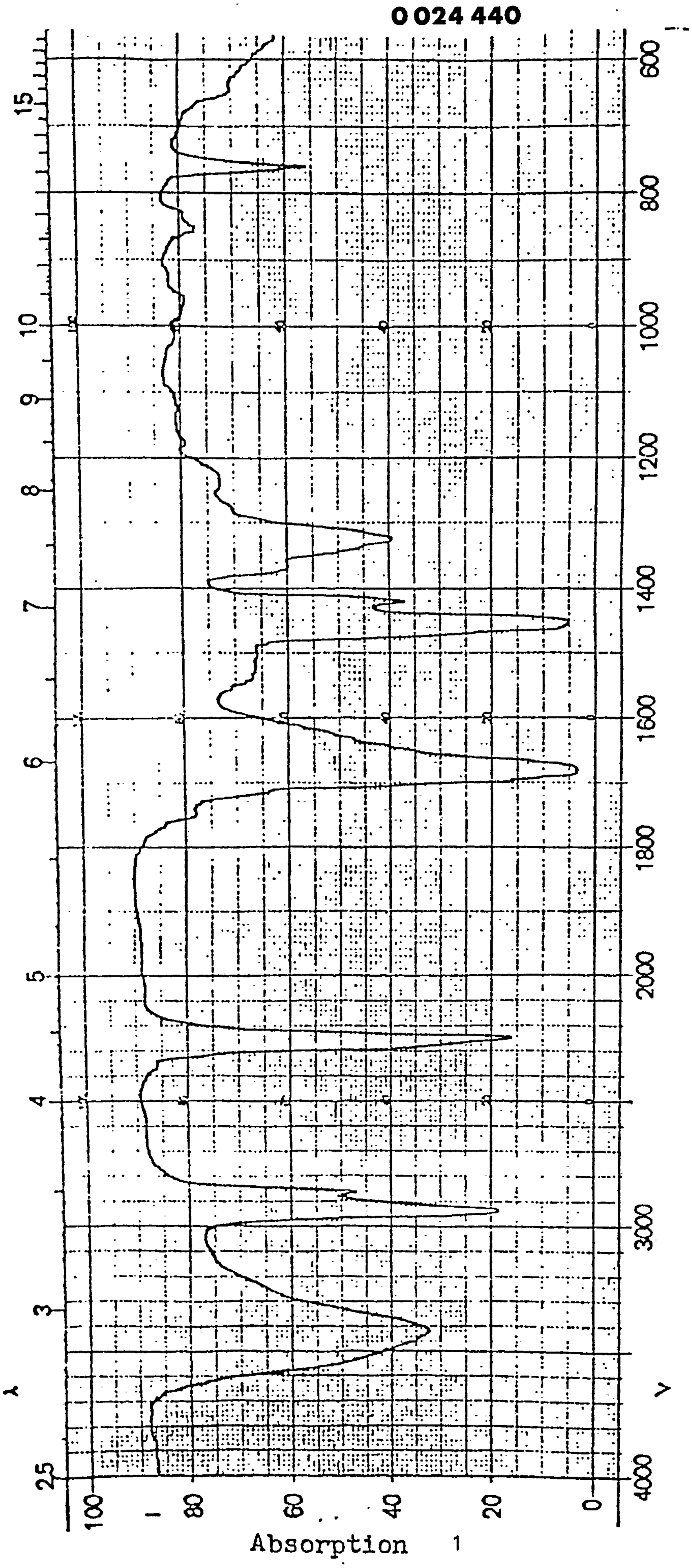

Abb. 2
VEBA-CHEMIE AG